# EUROPÄISCHE PATENTSCHRIFT

(11) **EP 0 378 107 B1**
(45) Veröffentlichungstag und Bekanntmachung des Hinweises auf die Patenterteilung: **08.12.1993**
(21) Anmeldenummer: 90100150.3
(22) Anmeldetag: 04.01.1990
(51) Int. Cl.: A61L 2/00

(54) **Verfahren zur Herstellung virusfreier Naturstoffe**
Method of preparing natural products free of viruses
Méthode de préparation de produits naturels sans virus

(30) Priorität: 07.01.1989 DE 3900350
(43) Veröffentlichungstag der Anmeldung: 18.07.1990
(73) Patentinhaber: Dr. Karl Thomae GmbH, D-88397 Biberach (DE)
(72) Erfinder: Disse, Bernd, Dr. Dr. Dipl.-Chem., D-7950 Biberach 1 (DE); Weller, Eberhard, D-7950 Biberach 1 (DE)

(56) Entgegenhaltungen:
- EP-A- 0 083 999
- EP-A- 0 112 563
- WO-A-84/01894
- WO-A-85/05273

## Beschreibung

Die Erfindung betrifft ein verfahren zur Herstellung eines von aktiven unbehüllten Viren freien Naturstoffs.

Lipidextraktionen mit halogenierten Kohlenwasserstoffen, wie Chloroform oder Freon, führen bekanntlicherweise zu einer Inaktivierung von behüllten Viren durch Zerstörung der Hülle (vgl. EP-Bl-0 111 549). Nicht behüllte Viren gelten bis heute als resistent gegenüber vielen organischen Lösungsmitteln und bilden damit möglicherweise eine Quelle zur Kontamination von Stoffen biologischer Herkunft, beispielsweise von Naturstoffen zur parenteralen Anwendung beim Menschen, wie Organextrakte oder Hormonpräparationen.

Von E.G. Bligh und W.J. Dyer wurde eine Methode zur Lipidextraktion mit Hilfe von Chloroform-Methanol-Wassergemischen beschrieben (vgl. Can. J. Biochem. Physiol. Vol. 37 (1959), 911-917). Eine optimale Lipidextraktion erzielt man dann, wenn das Gewebe mit einer Mischung aus Chloroform und Methanol homogenisiert wird, das mit dem im Gewebe enthaltenen Wasser eine monophasische Mischung ergibt. Anschließend kann das Homogenat mit Wasser und/oder Chloroform unter Bildung eines biphasischen Systems verdünnt werden, wobei die Chloroformphase die Lipide enthält und die Methanol-Wasser-Phase die Nichtlipide. Von einer Inaktivierung von Viren, insbesondere von nichtumhüllten Viren, ist in dieser Arbeit nichts ausgesagt.

In der EP-A-0 112 563 ist ein Verfahren zur Desaktivierung von Viren, die in ihrem strukturellen Aufbau Lipide enthalten, in Plasmaprotein-Produkten beschrieben. Das Verfahren besteht in der Behandlung des Produktes mit organischen Lösungsmitteln, in denen die strukturellen Lipide der viralen Verunreinigungen im Gegensatz zum Plasmaprotein löslich sind. Als Lösungsmittel werden unter anderem Chloroform, Ether, aromatische Kohlenwasserstoffe und kurzkettige Alkohole sowie deren Mischungen genannt. Ein Zusatz von Wasser wird nicht erwähnt.

In der WO 85/05273 ist ein Verfahren zur Desaktivierung von lipidhaltigen Viren, insbesondere von Hepatitis B und non-A, non-B Hepatitis Viren, in lyophilisierten Plasmaprotein-Produkten durch Behandlung des Produkts mit Lösungsmitteln, die bei der jeweiligen Behandlungstemperatur zu 75 bis 100% mit Wasser gesättigt sind, beschrieben. Als bevorzugte Lösungsmittel werden Chloroform, gegebenenfalls in Mischung mit einem kurzkettigen Alkohol wie Methanol oder Ethanol, sowie Fluorchlorkohlenwasserstoffe genannt.

Von einer desaktivierenden Wirkung auf nicht umhüllte Viren wird in den beiden letztgenannten Patentschriften nichts erwähnt.

Überraschenderweise wurde nun gefunden, daß nicht behüllte Viren, die gegenüber halogenhaltigen aliphatischen Kohlenwasserstoffen, wie Chloroform und Alkoholen, wie Methanol, allein jeweils resistent sind, mit einem Gemisch bestehend aus einem halogenhaltigen aliphatischen Kohlenwasserstoff und einem Alkohol bis zu 6 Kohlenstoffatomen abgetötet bzw. inaktiviert werden. Zu diesem Zwecke haben sich Gemische aus vorzugsweise Chloroform und Äthanol und/oder Propanol und/oder Butanol mit/oder ohne Wasserzusatz als geeignet erwiesen, besonders aber Gemische aus Chloroform und Methanol. Besonders gute Resultate liefert ein Methanol-Chloroform-Wasser-Gemisch, vorzugsweise in Form seiner ternären Phase.

Als halogenhaltige aliphatische Kohlenwasserstoffe kommen z. B. 1,2-Dichlorethan, 1,1-Dichlorbutan, 1,1,1-Trichlorethan, Trichlorethylen insbesondere aber Chloroform, als Alkohole z. B. Ethanol, Propanol, Isopropanol, n-Butanol, n-Hexanol, insbesondere aber Methanol in Frage. Ein Wasserzusatz kommt besonders dann in Frage, wenn sich damit ternäre Phasen erzielen lassen.

Die Inaktivierung bzw. Abtötung gelingt im allgemeinen durch Homogenisierung oder Auflösen oder bloße Behandlung des entsprechenden Substrats bzw. Materials in bzw. mit einem Gemisch bestehend aus dem jeweiligen halogenhaltigen aliphatischen Lösungsmittel und einem Alkohol und, gegebenenfalls, Wasser, beispielsweise bestehend aus Wasser bzw. physiologischer Kochsalzlösung, Chloroform und Methanol, wobei das Gemisch oder Gemenge für etwa 1 bis 3 Stunden bei niederen Temperaturen, beispielsweise bei -20 bis 10°C, gelagert wird. Besonders vorteilhaft ist die Behandlung oder Auflösung des Materials in einer homogenen Phase bestehend aus einem Volumenteil physiologischer Kochsalzlösung, 1,1 Volumenteilen Chloroform und 2,2 Volumenteilen Methanol, wobei nach einer Einwirkzeit von z.B. 2 Stunden ein weiterer Volumenteil Kochsalzlösung und 1,1 Volumenteile Chloroform zugefügt werden. Es tritt eine Trennung in zwei Phasen ein. Sowohl in der wässrigen als auch in der organischen Phase ist keine Virusaktivität unbehüllter Viren mehr nachweisbar.

Das gefundene Verfahren zur Herstellung eines von aktiven unbehüllten Viren freien Naturstoffs läßt sich für die Aufarbeitung aller natürlicher Rohstoffe, die für eine Anwendung beim Menschen oder bei Tieren in Frage kommen, verwenden. Das Verfahren kommt nicht nur für lipidextrahierbare Materialien sondern auch für lediglich lösemittelresistente Stoffe in Betracht, da auch z. B. die wässrige Phase keine Virusaktivität mehr aufweist.

Die Durchführbarkeit des erfindungsgemäßen Verfahrens soll anhand eines Beispiels demonstriert werden. Dieses Beispiel bezieht sich auf das Surfactant SF-RI l, das aus der Lavage von gesunden, tierärztlich freigegebenen Rinderlungen durch Auswaschen der Alveolen mit physiologischer Kochsalzlösung in Anwesenheit organischer Lösungsmittel, z. B. Chloroform hergestellt wird. SF-RI I ist ein Gemisch aus Phospholipiden (ca. 90 Gew.-%), Cholesterol (ca. 3 Gew.-%), Glyceriden (ca. 4 Gew.-%), Fettsäuren (ca. 0,3 Gew.-%) und Surfactant-assoziierten Proteinen des Typs B und C (ca. 1 Gew.-%). Im allgemeinen besteht keine Kontamination der Lungen mit rinderspezifischen Viren, eine Kontamination ist aber trotzdem nicht völlig auszuschließen. Um aber das erfindungsgemäße Verfahren auf seine Wirksamkeit testen zu können, wurde dem aus Lavage durch Zentrifugation gewonnenen Rohsurfactant ein unbehülltes Testvirus (ECBO-Virus, Stamm LCR-4 der Universität Gießen, Institut für Virologie der Vet. Med. Fakultät) zugesetzt. Dieses Testvirus ist repräsentativ für die bei Rindern relativ selten vorkommenden unbehüllten Viren.

Rinderbestände können z. B. von Maul- und Klauenseuche (Picorna-Virus) befallen sein. Zur Prüfung eines Surfactants auf Kontamination mit unbehüllten Viren müßte auf jedes eventuell in Frage kommende Virus getestet werden. Da dies kaum möglich ist, wurden dem zu untersuchenden Material vor der Aufarbeitung exemplarisch ausgesuchte Testviren zugefügt und dieses Material nach der erfindungsgemäßen Inaktivierung wiederum auf die Virenaktivität hin untersucht.

Als Testvirus wurde ein nicht behülltes RNS-Virus eingesetzt.

Als RNS-Virus diente das schon oben genannte ECBO-Virus, welches als offizieller Prüfstamm der DVG (Deutsche Veterinärmedizinische Gesellschaft) im Rahmen der Prüfung chemischer Desinfektionsmittel ausgewählt wurde; Genus: Enterovirus, Familie: Picornaviridae; einsträngiges RNS-Virus, unbehüllt, kubisches Kapsid, stabil gegenüber Chloroform, Durchmesser 24-30 nm.
Die Testviren wurden als Kulturüberstände mit 10⁵ KID₅₀/ml (BHV-1) bzw. 10^{6,8} KID₅₀/ml bezogen. Die Filter wurden vor Gebrauch autoklaviert (KID₅₀ bedeutet kultur-infektiöse Dosis an Viren, bei der 50 % der Zellen mit Viren infiziert sind).

Eine Validierungsstudie (Schema 2) wurde parallel zum Herstellungsprozeß mit dem Testvirus getrennt durchgeführt. Das Schema des Herstellungsprozesses, die Zugabe des Testvirus und die Entnahme der Proben sind in Schema 1 an Hand der Aufarbeitung des Rohsurfactants aus der Lavage von Rinderlungen aufgezeichnet:

### Schema 1:

Als Positivkontrolle wurde Viruskulturüberstand 1 + 1 mit 9 g/l Kochsalzlösung vermischt (Probe 1). Nach Filtration durch ein 0,22 µm-Filter wurde die Probe 1a gezogen. Diese Probe ersetzt das eigentlich zu prüfende kontaminierte Ausgangsmaterial, weil natives Surfactant nicht sterilfiltriert werden kann, da seine Teilchen größer als die Bakterien sind und damit das Filter verstopfen.

Aus Lavage von Rinderlungen wird das Rohsurfactant durch Zentrifugation gewonnen. Das erhaltene feuchte Pellet enthält neben Surfactant Zelltrümmer und Bakterien. An dieser Stelle wurden aus dem Produktionsprozeß 23 ml feuchtes Surfactant-Pellett entnommen und in 23 ml des virushaltigen Kulturüberstands suspendiert.

Diese kontaminierte Surfactantaufschwemmung wurde nach Bligh und Dyer extrahiert. Hierzu wurden 50,6 ml Chloroform und 101,2 ml Methanol hinzugegeben und die entstehende homogene organisch-wässrige Mischphase für 2 Stunden gekühlt gelagert, zweckmäßigerweise bei -10 bis -20°C. Die aufgetretene Proteinfällung wurde abzentrifugiert. Durch Zusatz von 46 ml 9 g/l Kochsalzlösung und 50,6 ml Chloroform wurde eine Phasentrennung herbeigeführt. Die wässrige Phase wurde abgetrennt und durch ein 0,22 µm Membranfilter sterilfiltriert (Probe 2). Die organische Phase wurde ebenfalls durch ein 0,22 µm Filter sterilfiltriert. Das Lösemittel wurde im Vakuum bei 10°C abdestilliert und das Lipid bei Raumtemperatur getrocknet. Pro 50 mg trockenes Lipid wurden 0,95 ml destilliertes Wasser zugesetzt. Durch ca. 10 minütiges Schütteln erhielt man Schüttelliposomen mit einer Vesikelgröße von ca. 1000 nm (Probe 3). Ein Teil der Schüttelliposomen wurde für 4 Minuten mit Ultraschall von 20 Watt Leistung beschallt (Branson Sonifier, Mikrospitze). Hierbei entstanden Vesikel von 200 nm (Probe 4).

### Schema 2:

Zur Absicherung des gefundenen viruziden Prinzips wurde der Einfluß einzelner Prozeßschritte näher untersucht.

Hierzu wurden 4 ml virushaltiger Kulturüberstand (ECBO-Virus) mit 4 ml 9 g/l Kochsalzlösung vermischt und sterilfiltriert (Probe 1).

5 ml Virussuspension (ECBO-Virus) wurden mit 1 ml Chloroform geschüttelt. Die wässrige Phase wurde abgetrennt und sterilfiltriert (Probe 2).

5 ml Virussuspension (ECBO-Virus) wurden mit 0,5 ml Methanol vermischt und sterilfiltriert (Probe 3).

5 ml Virussuspension (ECBO-Virus) wurden mit 11 ml Methanol und 5,5 ml Chloroform zu einer homogenen Phase vermischt. Durch Zugabe von 5 ml 9 g/l Kochsalzlösung und 5,5 ml Chloroform wurde die Phasentrennung bewirkt. Die wässrige Phase wurde sterilfiltriert (Probe 4).

1 g SF-RI l-Lipid wurde in einer Mischung aus 5 ml Virussuspension (ECBO-Virus) mit 11 ml Methanol und 5,5 ml Chloroform gelöst. Nach Zugabe von 5 ml 9 g/l Kochsalzlösung und 5,5 ml Chloroform erfolgte die Phasentrennung. Die wässrige Phase wurde abgetrennt und sterilfiltriert (Probe 5).

Alle Proben wurden auf MDBK-Zellkulturen (Nadin und Darby, Bovine Kidney: ATCC CCL 22) im Adsorptionsverfahren und per Inokulation in das Kulturmedium untersucht. Alle Proben außer 1 (1a) wurden wegen zelltoxischer Eigenschaften erst nach mehreren Subpassagen auf ECBO-Virus - spezifische Veränderungen untersucht.

Durch Vermischung des Surfactant-Rohmaterials im Verhältnis 1 + 1 mit Kulturüberstand (10⁵ und 10^{6,8} KID₅₀/ml) wurde eine hohe Kontamination des Ausgangsmaterials simuliert. Die Nachweisgrenze für die benutzten Testviren beträgt ca. 10 KID₅₀/ml. Nur in den Proben 1 und 1a nach Schema 1 konnte das unbehüllte ECBO-Virus nachgewiesen werden. In den Proben 3 und 4 (Schema 1), die jeweils Entnahme aus dem Produktionsprozeß zu verschiedenen Zeitpunkten simulieren, konnte das Testvirus nicht nachgewiesen werden. Die Studien nach Schema 1 zeigen, daß bereits beim Extraktionsverfahren nach Bligh und Dyer die zugesetzten Testviren inaktiviert oder abgetrennt wurden. Sowohl in der wässrigen Phase (Probe 2) als auch in der Trockenmasse aus der organischen Phase (Proben 3 und 4) konnte das Testvirus nicht mehr nachgewiesen werden.

Dieser Befund war für das unbehüllte ECBO-Virus nicht vorhersehbar.

Zur näheren Eingrenzung und weiteren Absicherung des Virusinaktivierenden Teilschritts wurden nach Schema 2 ECBO-Viren mit Chloroform, Methanol, Methanol-Chloroform-Kochsalzlösung, sowie mit Chloroform-Methanol-Kochsalzlösung-SF-RI l versetzt. In der Positivkontrolle und den Proben mit Chloroform oder Methanol (Proben 1 bis 3) konnten die cytopathogenen Effekte des ECBO-Virus nachgewiesen werden. Die Proben 4 und 5 zeigten hingegen keine virusspezifischen Effekte auf MDBK-Zellkulturen.

Dieses Ergebnis zeigt, daß die organischen Lösemittel Chloroform oder Methanol allein ECBO-Viren nicht inaktivieren und auch das Testergebnis nicht beeinträchtigen. Erst die Einwirkung von Chloroform und Methanol (hier in homogener organisch-wässriger Mischphase) inaktivierte das nicht umhüllte Testvirus.

Wie dieses Testmodell aufzeigt eignet sich das erfindungsgemäße Verfahren zur Entfernung bzw. Inaktivierung von nicht behüllten Viren in organischen Präparaten bzw. Präparationen.

## Patentansprüche

1. Verwendung eines Gemisches aus halogenhaltigen aliphatischen Kohlenwasserstoffen, Alkoholen mit bis zu 6 Kohlenstoffatomen und Wasser zur Inaktivierung von nicht umhüllten Viren in Naturstoffen.

2. Verwendung eines Gemisches aus Chloroform und Methanol und/oder Äthanol und/oder Propanol und/oder Butanol und Wasser gemäß Anspruch 1.

3. Verwendung einer ternären Phase aus Chloroform, Methanol und Wasser gemäß Anspruch 1.

4. Verwendung eines Gemisches gemäß den Ansprüchen 1 bis 3, wobei anstelle von Wasser eine physiologische Kochsalzlösung zugemischt wird.

5. Verwendung eines Gemisches gemäß den Ansprüchen 1 bis 4 zur Inaktivierung von Viren der Familie Picornaviridae in Naturstoffen.

6. Verwendung eines Gemisches gemäß den Ansprüchen 1 bis 4 zur Inaktivierung von ECBO-Viren in Naturstoffen.

7. Verwendung gemäß den Ansprüchen 1 bis 4, dadurch gekennzeichnet, daß der Naturstoff ein aus Rinderlungen durch Lavage gewonnenes Surfactant ist.

8. Verfahren zur Herstellung eines von aktiven nicht umhüllten Viren freien Surfactants, bestehend aus Phospholipiden, Cholesterol, Glyceriden, Fettsäuren und Surfactant-assoziierten Proteinen des Typs B und C, dadurch gekennzeichnet, daß ein aus der Lavage von Rinderlungen durch Auswaschen der Alveolen mit physiologischer Kochsalzlösung gewonnenes Lungensurfactant extrahiert wird, wobei unter Zugabe von Chloroform und Methanol eine homogene organisch-wäßrige Mischphase entsteht, eine gleichzeitig auftretende Proteinfällung abgetrennt wird, anschließend durch Zugabe von Kochsalzlösung und Chloroform eine Phasentrennung herbeigeführt wird und das Surfactant aus der organischen Phase isoliert wird.

9. Surfactant, dadurch gekennzeichnet, daß es durch ein Verfahren gemäß Anspruch 8 hergestellt wird.

## Claims

1. Use of a mixture of halogen-containing aliphatic hydrocarbons, alcohols having up to 6 carbon atoms and water, for inactivating non-enveloped viruses in natural substances.

2. Use of a mixture of chloroform and methanol and/or ethanol and/or propanol and/or butanol and water according to claim 1.

3. Use of a ternary phase of chloroform, methanol and water according to claim 1.

4. Use of a mixture according to claims 1 to 3, in which a physiological saline solution is added instead of water.

5. Use of a mixture according to claims 1 to 4 for inactivating viruses of the Picorna viridae family in natural substances.

6. Use of a mixture according to claims 1 to 4 for inactivating ECBO viruses in natural substances.

7. Use according to claims 1 to 4, characterised in that the natural substance is a surfactant obtained from cattle lungs by washing out.

8. Process for preparing a surfactant free from active non-enveloped viruses, consisting of phospholipids, cholesterol, glycerides, fatty acids and surfactant-associated proteins of type B and C, characterised in that a lung surfactant is extracted, which is obtained by washing out cattle lungs by washing the alveolae with physiological saline solution, wherein a homogeneous, organic-aqueous mixed phase is produced by adding chloroform and methanol, a protein precipitate formed at the same time is separated off, then phase separation is effected by the addition of saline solution and chloroform and the surfactant is isolated from the organic phase.

9. Surfactant, characterised in that it is prepared by a process according to claim 8.

## Revendications

1. Utilisation d'un mélange d'hydrocarbures aliphatiques halogénés, d'alcools ayant jusqu'à 6 atomes de carbone et d'eau pour inactiver des virus sans enveloppe dans des substances naturelles.

2. Utilisation d'un mélange de chloroforme et de méthanol et/ou d'éthanol et/ou de propanol et/ou de butanol et d'eau selon la revendication 1.

3. Utilisation d'une phase ternaire de chloroforme, de méthanol et d'eau selon la revendication 1.

4. Utilisation d'un mélange selon les revendications 1 à 3, dans lequel une solution physiologique de chlorure de sodium est introduite à la place de l'eau.

5. Utilisation d'un mélange selon les revendications 1 à 4 pour l'inactivation des virus de la famille des picornaviridés dans des substances naturelles.

6. Utilisation d'un mélange selon les revendications 1 à 4 pour l'inactivation des virus ECBO dans des substances naturelles.

7. Utilisation selon les revendications 1 à 4, caractérisée en ce que la substance naturelle est un tensioactif obtenu à partir de poumons de boeuf par lavage.

8. Procédé de préparation d'un tensioactif exempt de virus sans enveloppe actifs, consistant en phospholipides, en cholestérol, en glycérides, en acides gras et en protéines de type B et C associées au tensioactif, caractérisé en ce qu'un tensioactif pulmonaire obtenu à partir du lavage de poumons de boeuf par lavage des alvéoles avec une solution physiologique de chlorure de sodium est extrait, une phase mixte organique-aqueuse homogène se forme par addition de chloroforme et de méthanol, un précipité protéique formé simultanément est séparé, puis une séparation de phases est réalisée par addition de solution de chlorure de sodium et de chloroforme et le tensioactif est isolé à partir de la phase organique.

9. Tensioactif caractérisé en ce qu'il est préparé par un procédé selon la revendication 8.
